(19) Europäisches Patentamt
European Patent Office
Office européen des brevets

(11) Veröffentlichungsnummer: **0 177 448**
**B1**

(12) # EUROPÄISCHE PATENTSCHRIFT

(45) Veröffentlichungstag der Patentschrift: **03.05.89**

(51) Int. Cl.⁴: **C 07 D 327/06**, C 07 D 411/12, C 07 D 307/79

(21) Anmeldenummer: **85810437.5**

(22) Anmeldetag: **25.09.85**

(54) **Verfahren zur Herstellung von 1,2-Benzoxathiin-Derivaten.**

(30) Priorität: **01.10.84 US 656193**

(43) Veröffentlichungstag der Anmeldung: **09.04.86 Patentblatt 86/15**

(45) Bekanntmachung des Hinweises auf die Patenterteilung: **03.05.89 Patentblatt 89/18**

(84) Benannte Vertragsstaaten: **AT BE CH DE FR GB IT LI NL**

(56) Entgegenhaltungen:
**INT. J. SULFUR CHEM.,A, Band 2, Nr. 4, 1972, Seiten 249-255; J.M. CLANCY et al.: "Synthesis and mass spectrometry of sultones"**

(73) Patentinhaber: **CIBA-GEIGY AG, Klybeckstrasse 141, CH-4002 Basel (CH)**

(72) Erfinder: **Föry, Werner, Dr., Benkenstrasse 65, CH-4054 Basel (CH)**

EP 0 177 448 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 3,4-Dihydro-2,2-dioxo-3-methyl-1,2-benzoxathiin-8-yl-sulfonamid.

Das nach dem erfindungsgemässen neuen Verfahren herstellbare 3,4-Dihydro-2,2-dioxo-3-methyl-1,2-benzoxathiin-8-ylsulfonamid ist ein wertvolles Zwischenprodukt für die Herstellung von Herbiziden und Pflanzenwuchsregulatoren der Klasse der Sulfonylharnstoffe. Solche Wirkstoffe sind mit ihren biologischen Eigenschaften beispielsweise aus der publizierten Europäischen Patentanmeldung EP-A-99 339 bekannt.

Die Erzeugung von Verbindungen mit 1,2-Benzoxathiin-Struktur ist schon verschiedentlich beschrieben worden: Int. J. Sulfur Chem., A. Volume 2, No. 4 249–255 (1972) oder EP-A 107 979. Die angewendeten Verfahren erweisen sich für den grosstechnischen Einsatz als wenig geeignet, da sie über eine relativ grosse Anzahl von Reaktionsstufen verlaufen.

Es besteht daher ein Bedürfnis nach einem einfachen, wenige Reaktionsschritte umfassenden Syntheseverfahren, das die Herstellung des gewünschten Zwischenproduktes in guter Ausbeute erlaubt.

Überraschenderweise wurde nun festgestellt, dass es gelingt, 3,4-Dihydro-2,2-dioxo-3-methyl-1,2-benzoxathiin-8-ylsulfonamid der Formel I

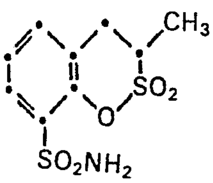

(I),

herzustellen, indem man ein 5-Halogen-2,3-dihydro-2-methyl-benzo[b]-furan der Formel II

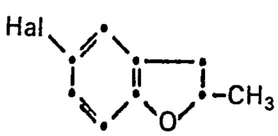

(II),

worin Hal für Chlor, Brom oder Jod steht, mit Chlorsulfonsäure zu einem 6-Halogen- 2,2-dioxo-3-methyl-1,2-benzoxathiin-8-ylsufonylchlorid der Formel III

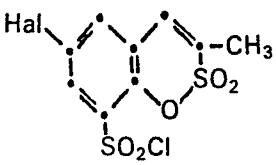

(III),

worin Hal für Chlor, Brom oder Jod steht, umsetzt, dieses mit Ammoniak in ein 6-Halogen-2,2-dioxo-3-methyl- 1,2-benzoxathiin- 8-yl-sulfonamid der Formel IV

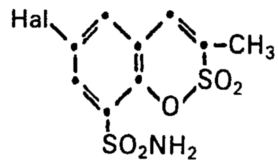

(IV),

worin Hal für Chlor, Brom oder Jod steht, überführt, dieses in Gegenwart eines tertiären Amins und eines Edelmetallkatalysators mit Wasserstoff dehalogeniert und das entstehende 2,2-Dioxo-3-methyl- 1,2-benzoxathiin- 8-yl-sulfonamid der Formel V

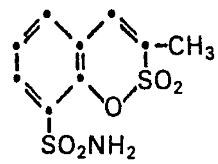

(V)

in Gegenwart eines Edelmetallkatalysators mit Wasserstoff hydriert.

Im Rahmen der vorliegenden Erfindung steht Hal für Chlor, Brom oder Jod. Bevorzugt sind Chlor und Brom.

Das Reaktionsprodukt der Formel I kann in bekannter Weise direkt oder in Form des entsprechenden Isocyanats oder eines Carbamats zu einem Pflanzenwirkstoff aus der Klasse der Sulfonylharnstoffe umgesezt werden.

Die Ausgangsverbindungen der Formel II ist bekannt und kann durch Halogenierung von 2,3-Dihydro- 2-methylbenzo[b]furan erzeugt werden.

Zur Durchführung des ersten Schrittes (II→III) des erfindungsgemässen Verfahrens verwendet man handelsübliche Chlorsulfonsäure. Zur Umsetzung werden pro Mol an Verbindung II mindestens 3 Mol Chlorsulfonsäure eingesetzt. Vorteilhaft ist die Verwendung eines grösseren Überschusses, beispielsweise von mindestens 5 Mol Chlorsulfonsäure pro Mol der Verbindung II. In einzelnen Ausführungsformen kann die Chlorsulfonsäure gleichzeitig als Reaktionspartner und als Lösungsmittel dienen. Im allgemeinen wird die Umsetzung (II→III) jedoch in einem inerten Lösungsmittel durchgeführt. Bewährte Lösungsmittel sind dafür Schwefelkohlenstoff, Äthylacetat und chlorierte Kohlenwasserstoffe wie Methylenchlorid, Chloroform, Tetrachlorkohlenstoff, 1,2-Dichloräthan 1,2-Dichloräthylen, Tetrachloräthylen, Chlorbenzol oder Dichlorbenzol. Die bevorzugten Lösungsmittel sind Methylenchlorid, 1,2-Dichloräthan und Chloroform. Die Reaktionstemperaturen liegen meistens zwischen −10 °C, vorzugsweise zwischen −10 °C und +60 °C.

In einer bevorzugten Ausführungsform der ersten Reaktionsstufe (II→III) stellt man die Verbindungen der Formel III, worin Hal für Chlor oder Brom steht, her, indem man die entsprechende Verbindung der Formel II in einem inerten Lösungsmittel bei einer Temperatur zwischen −10 °C und +80 °C mit mindestens 3 Mol Chlorsulfonsäure pro Mol der Verbindung der Formel II

umsetzt. Ganz besonders bevorzugt ist diejenige Ausführungsform, in welcher man die Reaktion (II→III) bei einer Temperatur zwischen −10 °C und +60 °C in Methylenchlorid 1,2-Dichloräthan oder Chloroform mit mindestens 5 Mol Chlorsulfonsäure pro Mol der Verbindung der Formel II durchführt.

Die Überführung des Sulfonylchlorids der Formel III in das entsprechende Sulfonamid der Formel IV erfolgt unter den für diesen an sich bekannten Reaktionsschritt üblichen Bedingungen, beispielsweise durch Versetzen der Verbindung der Formel III mit einer wässrigen Ammoniak-Lösung unter Normaldruck und bei Raumtemperatur; oder durch Behandlung einer Verbindung der Formel III in einem inerten Lösungsmittel, gegebenenfalls in Gegenwart eines säurebindenden Mittels, mit Ammoniak. Lösungsmittel und säurebindende Mittel sind beispielsweise: Carbonate wie Natrium- und Kaliumcarbonat, Hydrogencarbonate wie Natrium- und Kaliumhydrogencarbonat, Oxide wie Calcium- und Magnesiumoxid oder Hydroxide wie Natrium-, Kalium-, Calcium- oder Magnesiumhydroxid; Äther wie Diäthyläther, Tetrahydrofuran, Dioxan, Äthylenglykoldimethyläther oder Diäthylenglykoldimethyläther; oder Kohlenwasserstoffe wie Cyclohexan, Benzol, Toluol oder Xylol. Bevorzugt ist die Umsetzung mit wässriger Ammoniak-Lösung.

Die Dehalogenierung der Verbindung der Formel IV zur Verbindung der Formel V durch katalytische Hydrierung wird im allgemeinen unter milden Bedingungen bei Raumtemperatur unter Normaldruck in einem inerten Lösungsmittel in Gegenwart eines säurebindenden Mittels mit Wasserstoff durchgeführt. Als Katalysatoren werden im wesentlichen Edelmetallkatalisatoren verwendet wie Platin oder Palladium in Form von Platinoxid, Platinmoor oder Platin auf Bariumsulfat, Palladiummoor oder Palladium auf Kohle. Am breitesten einsetzbar ist Palladium auf Kohle in handelsüblicher Form als 5% Palladium/Kohle. Als säurebindende Mittel werden üblicherweise verwendet: Carbonate wie Natrium-, Kalium- oder Calciumcarbonat, Hydrogencarbonat, Oxide wie Magnesium- oder Calciumoxide sowie vorzugsweise tertiäre organische Amine wie Trimethylamin, Triäthylamin, Diazabicyclo [2.2.2.]octan, 1,8-Diazabicyclo[5.4.0]undec-7-en, 1,5-Diazabicyclo[4.3.0]non-5-en, Pyridin, Chinolin oder Isochinolin. Als Lösungsmittel kommen in Frage: Äther wie Diätyläther, Tetrahydrofuran oder Dioxan; Ester wie Äthylacetat; Alkohole wie Methanol, Äthanol, n-Propanol oder 1-Propropanol und Kohlenwasserstoffe wie Pentan, Hexan, Cyclohexan, Benzol, Toluol oder Xylol. In der bevorzugten Ausführungsform wird die Verbindung der Formel IV unter Normaldruck bei 20 °C bis 25 °C in Gegenwart eines 5%igen Palladium/Kohle-Katalysators in Tetrahydrofuran mit Wasserstoff hydriert.

Die katalytische Hydrierung der nichtaromatischen Doppelbindung der Verbindung V im letzten Reaktionsschritt (V → I) erfolgt gegenüber dem vorherigen Reaktionsschritt (IV → V) unter verschärften Reaktionsbedingungen, das heisst sowohl der Druck der Wasserstoffatmosphäre als auch die Temperatur werden mit Vorteil erhöht. Bevorzugte Bedingungen sind 1 bis 10 bar und 30 °C bis 60 °C. Der Katalysator und das Lösungsmittel werden aus der gleichen Gruppe gewählt wie bei der vorherigen Reaktionsstufe. In der bevorzugten Ausführungsform wird die Verbindung V in Tetrahydrofuran bei 30 °C bis 50 °C unter 1 bis 5 bar in Gegenwart von 5%igem Palladium/Kohle-Katalysator hydriert.

In einer bevorzugten Ausführungsform des erfindungsgemässen Verfahrens zur Herstellung der Verbindungen der Formel I setzt man eine Verbindung der Formel II, worin Hal für Brom oder Chlor steht, bei einer Temperatur zwischen −10 °C und +60 °C in Methylenchlorid, 1,2-Dichloräthan oder Chloroform mit mindestens 5 Mol Chlorsulfonsäure pro Mol der Verbindung der Formel II um, versetzt das entstandene Sulfonylchlorid der Formel III mit wässriger Ammoniak-Lösung, dehalogeniert das erhaltene Sulfonamid der Formel IV durch katalytische Hydrierung in Tetrahydrofuran mit Wasserstoff unter Normaldruck bei einer Temperatur von 20 °C bis 25 °C in Gegenwart von 5%igem Palladium/Kohle-Katalysator und hydriert das dehalogenierte Sulfonamid der Formel V in Tetrahydrofuran bei 30° bis 50 °C unter einem Druck von 1 bis 5 bar in Gegenwart von 5%igem Palladium/Kohle-Katalysator.

Die folgenden Beispiele dienen der näheren Illustration der vorliegenden Erfindung. Das Beispiel H3 ist als Variation des zweiten Reaktionsschrittes des Beispiels H1 zu verstehen.

Herstellungsbeispiele:

Beispiel H1:

3,4- Dihydro -2,2- dioxo -3- methyl -1,2-benzoxathiin-8-yl-sulfonamid.

a) 5-Brom-2,3-dihydro-2-methylbenzo [b]furan.

Zu einer eisgekühlten Mischung von 562,3 g 2,3-Dihydro- 2-methylbenzo[b]furan, 1600 ml Methylenchlorid, 1600 ml Wasser und 352,5 g Natriumhydrogencarbonat lässt man innerhalb von 2,5 Stunden eine Lösung von 215 ml Brom in 450 ml Methylenchlorid zutropfen. Nachdem das Gemisch für weitere 1,5 Stunden bei der gleichen Temperatur gerührt worden ist, wird die wässrige Phase abgetrennt und zweimal mit je 300 ml Methylenchlorid extrahiert. Die vereinigten organischen Phasen werden zweimal mit je 250 ml Wasser gewaschen, über Natriumsulfat getrocknet und eingedampft. Durch Abtrennen aller Bestandteile mit einem Siedepunkt unterhalb von 99 °C bei 8 mbar erhält man als Rückstand 750,7 g 5-Brom-2,3-dihydro-2-methylbenzo[b]furan.

b) 6- Brom -2,2-dioxo -3-methyl -1,3-benzoxathiin -8-ylsulfonylchlorid

Zu einer auf −7 °C gekühlten Lösung von 160 g 5-Brom-2,3-dihydro-2-methylbenzo[b]furan in 460 ml absolutem Chloroform lässt man innerhalb von 20 Minuten 400 ml Chlorsulfonsäure zutropfen. Die Mischung wird bei einer Temperatur von 15 °C für 15 Minuten gerührt und anschliessend innerhalb von 35 Minuten tropfenweise in eine Mischung von 1,5 kg Eis, 1000 ml Wasser und 500 ml Chloroform eingerührt. Nachdem dieses Gemisch für 15 Minuten bei 0 °C gerührt worden ist, wird die organische Phase abgetrennt und die wässrige dreimal mit je 250 ml Chloroform extrahiert. Die vereinigten organischen Phasen werden mit 150 ml Wasser gewaschen und über Natriumsulfat getrocknet. Durch Eindampfen erhält man als öligen Rückstand, der direkt weiterverarbeitet werden kann, 180 g 6-Brom -2,2-dioxo -3-methyl -1,2-benzoxathiin -8-ylsulfonylchlorid.

c) 6- Brom -2,2-dioxo -3-methyl -1,2-benzoxathiin -8-ylsulfonamid

Eine Lösung von 180 g 6-Brom-2,2-dioxo-3-methyl-1,2-benzoxathiin-8-ylsulfonylchlorid in 500 ml Tetrahydrofuran wird innerhalb von 30 Minuten tropfenweise zu 307 ml einer 30%igen wässrigen Ammoniaklösung zugesetzt. Das Gemisch wird für 30 Minuten bei 20 °C gerührt und unter vermindertem Druck bei 45 °C eingedampft. Der Rückstand wird mit 200 ml Äther verrieben. Der ausgefallene Niederschlag wird abgetrennt, mit Wasser gewaschen und bei einer Temperatur von 45 °C getrocknet. Man erhält so 63,2 g 6-Brom -2,2-dioxo -3-methyl -1,2-benzoxathiin-8-ylsulfonamid, Smp. 243–245 °C (aus Äthanol).

d) 2,2-Dioxo -3-methyl -1,2-benzoxathiin -8-ylsulfonamid

120 g 6-Brom-2,2-dioxo-3-methyl-1,2-benzoxathiin-8-ylsulfonamid werden in 2,4 l Tetrahydrofuran gelöst und in Gegenwart von 41,3 g Triäthylamin und 12,0 g 5%igem Palladium/Kohle-Katalysator unter Normaldruck bei einer Temperatur zwischen 20 °C und 25 °C innerhalb von 40 Minuten mit Wasserstoff hydriert. Das Gemisch wird filtriert, das Filtrat eingedampft und der Rückstand in 1300 ml heissem 90% wässrigem Äthanol aufgenommen. Die unlöslichen Bestandteile werden abgetrennt, die Lösung auf 0 °C abgekühlt. Der ausgefallene Niederschlag wird abgetrennt und getrocknet. Man erhält 70 g 2,2-Dioxo- 3-methyl- 1,2-benzoxathiin-8-ylsulfonamid, Smp. 204–205 °C.

e) 10,0 g 2,2-Dioxo-4-methyl-1,2-benzoxathiin-8-yl-sulfonamid werden in 200 ml Tetrahydrofuran gelöst und in Gegenwart von 2,0 g 5%igem Palladium/Kohle-Katalysator unter einem Druck von 4 bar und bei einer Temperatur von 40 °C innerhalb von 2,5 Stunden mit Wasserstoff hydriert. Nach dem Abtrennen des Katalysators wird die Lösung eingedampft und der Rückstand aus 120 ml 70%igem wässrigem Äthanol kristallisiert. Man erhält 9,0 g 3,4-Dihydro-2,2-dioxo- 3-methyl- 1,2-benzoxathiin- 8-ylsulfonamid, Smp. 185–186 °C.

Beispiel H2:

N-(3,4-Dihydro -2,2-dioxo-3-methyl -1,2-benzoxathiin -8-ylsulfonyl) -N'-(4-methoxy -6-methylpyrimidin -2-yl)-harnstoff

Eine Mischung aus 3,33 g 3,4-Dihydro -2,2-dioxo -3-methyl -1,2-benzoxathiin -8-ylsulfonamid, 1,84 ml 1,8-Diazabicyclo[5.4.0]undec-7-en, 3,2 g N-(4-Methoxy -6-methylpyrimidin -2-yl)phenylcarbamat und 35 ml absolutem Dioxan wird für 45 Minuten bei einer Temperatur zwischen 20 °C und 25 °C gerührt. Durch Eindampfen und Verreiben des öligen Rückstandes mit Äther und 14 ml 1N-Salzsäure erhält man einen kristallinen Niederschlag, den man abtrennt, mit Wasser wäscht und trocknet. Man erhält 4,96 g N-(3,4-Dihydro -2,2-dioxo -3-methyl -1,2- benzoxathiin -8-ylsulfonyl)-N'-(4-methoxy -6- methylpyrimidin -2-yl)-harnstoff Smp. 215–218 °C.

Beispiel H3:

6-Brom -2,2-dioxo -3-methyl -1,2-benzoxathiin -8-ylsulfonylchlorid.

Zu einer Lösung von 42,6 g 5-Brom-2,3-dihydro-2-methylbenzo[b]furan in 120 ml Chloroform lässt man bei einer Temperatur zwischen 40 °C und 50 °C innerhalb von 30 Minuten 133 ml Chlorsulfonsäure zutropfen. Das Gemisch wird für 4 Stunden zum Rückfluss erhitzt und nach dem Abkühlen auf 0 °C innerhalb von 30 Minuten tropfenweise einer Mischung von 400 g Eis, 500 ml Wasser und 100 ml Chloroform zugefügt. Die organische Phase wird abgetrennt, die wässrige dreimal mit je 100 ml Chloroform extrahiert. Die vereinigten organischen Phasen werden mit Wasser gewaschen und eingedampft. Man erhält als öligen Rückstand 72,2 g 6-Brom -2,2-dioxo -3-methyl -1,2-benzoxathiin -8- ylsulfonylchlorid.

**Patentansprüche**

1. Verfahren zur Herstellung von 3,4-Dihydro-2,2-dioxo-3-methyl-1,2-benzoxathiin-8-ylsulfonamid der Formel I

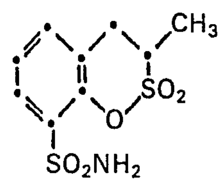

(I),

dadurch gekennzeichnet, dass man ein 5-Halogen- 2,3-dihydro- 2-methyl-benzo[b]furan der Formel II

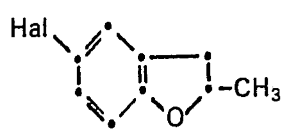

(II),

worin Hal für Chlor oder Brom steht, mit Chlorsulfonsäure zu einem 6-Halogen-2,2-dioxo-3-methyl-1,2-benzoxathiin-8-ylsulfonylchlorid der Formel III

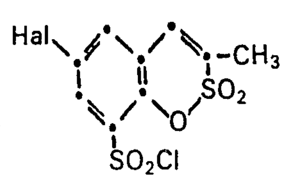

(III),

worin Hal für Chlor oder Brom steht, umsetzt, dieses mit Ammoniak in ein 6-Halogen-2,2-dioxo-3-methyl-1,2-benzoxathiin-8-yl-sulfonamid der Formel IV

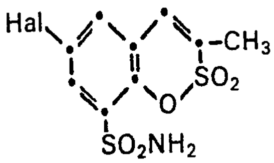

(IV),

worin Hal für Chlor oder Brom steht, überführt, dieses in Gegenwart eines tertiären Amins und eines Edelmetallkatalysators mit Wasserstoff dehalogeniert und das entstehende 2,2-Dioxo-3-methyl-1,2-benzoxathiin-8-yl-sulfonamid der Formel V

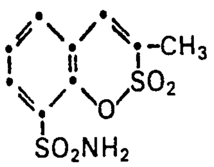

(V),

in Gegenwart eines Edelmetallkatalysators mit Wasserstoff hydriert.

2. Verfahren gemäss Anspruch 1, dadurch gekennzeichnet, dass Hal für Chlor oder Brom steht.

3. Verfahren gemäss Anspruch 1, dadurch gekennzeichnet, dass man die Reaktion in allen Reaktionsstufen bei Temperaturen zwischen −10 °C und +80 °C, vorzugsweise zwischen −10 °C und +60 °C, durchführt.

4. Verfahren gemäss Anspruch 1, dadurch gekennzeichnet, dass man die einzelnen Reaktionsstufen in inerten Lösungsmitteln durchführt.

5. Verfahren gemäss Anspruch 4, dadurch gekennzeichnet, dass man die Reaktion der Verbindung II zur Verbindung III in Methylenchlorid, 1,2-Dichloräthan oder Chloroform; die Reaktion der Verbindung III zur Verbindung IV in Wasser und die Reaktionen der Verbindung IV zur Verbindung V und der Verbindung V zur Verbindung I in Tetrahydrofuran ausführt.

6. Verfahren gemäss Anspruch 1, dadurch gekennzeichnet, dass man die Umsetzung der Verbindung der Formel II zur Verbindung der Formel III in einem inerten Lösungsmittel bei einer Temperatur zwischen −10 °C und +80 °C mit mindestens 3 Mol Chlorsulfonsäure pro Mol der Verbindung der Formel II durchführt.

7. Verfahren gemäss Anspruch 6, dadurch gekennzeichnet, dass man die Umsetzung der Verbindung der Formel II zur Verbindung der Formel III bei einer Temperatur zwischen −10 °C und +60 °C in Methylenchlorid, 1,2-Dichloräthan oder Chloroform mit mindestens 5 Mol Chlorsulfonsäure pro Mol der Verbindung der Formel II durchführt.

8. Verfahren gemäss Anspruch 1, dadurch gekennzeichnet, dass man die Umsetzung der Verbindung der Formel III zur Verbindung der Formel IV in wässriger Ammoniak-Lösung durchführt.

9. Verfahren gemäss Anspruch 1, dadurch gekennzeichnet, dass man die Dehalogenierung der Verbindung der Formel IV zur Verbindung der Formel IV unter Normaldruck bei 20 °C bis 25 °C in Gegenwart eines 5%igen Palladium/Kohle-Katalysators in Tetrahydrofuran mit Wasserstoff durchführt.

10. Verfahren gemäss Anspruch 1, dadurch gekennzeichnet, dass man die Hydrierung der Verbindung der Formel V zur Verbindung der Formel I in Tetrahydrofuran bei 30 °C bis 50 °C unter 1 bis 5 bar in Gegenwart von 5%igem Palladium/Kohle-Katalysator mit Wasserstoff durchführt.

11. Verfahren gemäss Anspruch 1, dadurch gekennzeichnet, dass man eine Verbindung der Formel II, worin Hal für Brom oder Chlor steht, bei einer Temperatur zwischen −10 °C und +60 °C in Methylenchlorid, 1,2-Dichloräthan oder Chloroform mit mindestens 5 Mol Chlorsulfonsäure umsetzt, das entstandene Sulfonylchlorid der Formel III mit wässriger Ammoniak-Lösung versetzt, das erhaltene Sulfonamid der Formel IV durch katalytische Hydrierung in Tetrahydrofuran mit Wasserstoff unter Normaldruck bei einer Temperatur von 20 °C bis 25 °C in Gegenwart von 5%igem Palladium/Kohle-Katalysator dehalogeniert und das dehalogenierte Sulfonamid der Formel V in Tetrahydrofuran bei 30 °C bis 50 °C unter einem Druck von 1 bis 5 bar in Gegenwart von 5%igem Palladium/Kohle-Katalysator hydriert.

## Revendications

1. Procédé de préparation de 3,4-dihydro-2,2-dioxo- 3-méthyl- 1,2-benzoxathiine-8-ylsulfonamide de formule I,

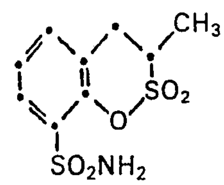

(I),

caractérisé en ce que l'on transforme un 5-halogéno-2,3-dihydro-2-méthyl-benzo[b]furanne de formule II

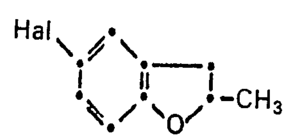

(II),

où Hal représente le chlore ou le brome, avec l'acide chlorosulfonique en un chlorure de 6-halogéno-2,2-dioxo-3-méthyl-1,2-benzoxathiine-8-ylsulfonyle de formule III

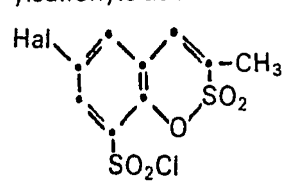

(III),

où Hal représente le chlore ou le brome, en ce que l'on transforme celui-ci avec l'ammoniaque en un

6-halogéno- 2,2-dioxo- 3-méthyl- 1,2-benzoxathiine-8-ylsulfonamide de formule IV

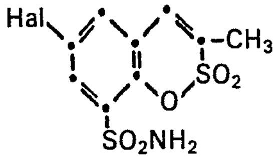
(IV),

où Hal représente le chlore ou le brome, en ce que l'on déshalogène celui-ci par l'hydrogène en présence d'une amine tertiaire et d'un catalyseur à base d'un métal noble et en ce que l'on hydrogène le 2,2-dioxo-3-méthyl-1,2-benzoxathiine-8-ylsulfonamide de formule V

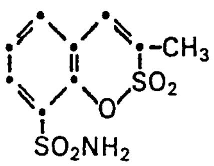
(V)

par l'hydrogène en présence d'un catalyseur à base d'un métal noble.

2. Procédé selon la revendication 1, caractérisé en ce que Hal représente le chlore ou le brome.

3. Procédé selon la revendication 1, caractérisé en ce que l'on effectue la réaction dans toutes les étapes réactionnelles à des températures comprises entre −10 °C et +80 °C, de préférence entre −10 °C et +60 °C.

4. Procédé selon la revendication 1, caractérisé en ce que l'on effectue les étapes réactionnelles individuelles dans des solvants organiques inertes.

5. Procédé selon la revendication 4, caractérisé en ce que l'on effectue la transformation du composé II en composé III dans le chlorure de méthylène, le 1,2-dichloroéthane ou le chloroforme; la transformation du composé III en composé IV dans l'eau et les transformations du composé IV en composé V et du composé V en composé I dans le tétrahydrofuranne.

6. Procédé selon la revendication 1, caractérisé en ce que l'on effectue la transformation du composé de formule II en composé de formule III dans un solvant inerte à une température entre −10 °C et +80 °C avec au moins 3 moles d'acide chlorosulfonique par mole du composé de formule II.

7. Procédé selon la revendication 6, caractérisé en ce que l'on effectue la transformation du composé de formule II en composé de formule III à une température entre −10 °C et +60 °C dans le chlorure de méthylène, le 1,2-dichloroéthane ou le chloroforme avec au moins 5 moles d'acide chlorosulfonique par mole de composé de formule II.

8. Procédé selon la revendication 1, caractérisé en ce que l'on effectue la transformation du composé de formule III en composé de formule IV dans une solution aqueuse d'ammoniaque.

9. Procédé selon la revendication 1, caractérisé en ce que l'on effectue la déshalogénation du composé de formule IV en composé de formule V par l'hydrogène sous la pression normale, à une température de 20 °C à 25 °C en présence d'un catalyseur palladium/charbon à 5% dans le tétrahydrofuranne.

10. Procédé selon la revendication 1, caractérisé en ce que l'on effectue l'hydrogénation du composé de formule V en composé de formule I par l'hydrogène dans le tétrahydrofuranne à une température de 30 °C à 50 °C sous 1 à 5 bar en présence d'un catalyseur palladium/charbon à 5%.

11. Procédé selon la revendication 1, caractérisé en ce que l'on fait réagir un composé de formule II où Hal représente le brome ou le chlore à une température entre −10 °C et +60 °C dans le chlorure de méthylène, le 1,2-dichloroéthane ou le chloroforme avec au moins 5 moles d'acide chlorosulfonique, en ce que l'on mélange le chlorure de sulfonyle obtenu de formule III avec une solution aqueuse d'ammoniaque, en ce que l'on déshalogène le sulfonamide obtenu de formule IV par hydrogénation catalytique dans le tétrahydrofuranne avec l'hydrogène sous la pression normale à une température de 20 °C à 25 °C en présence d'un catalyseur palladiun/charbon à 5% et en ce que l'on hydrogène le sulfonamide de formule V déshalogéné dans le tétrahydrofuranne à une température de 30 °C à 50 °C sous une pression de 1 à 5 bar en présence d'un catalyseur palladium/charbon à 5%.

## Claims

1. A process for the preparation of 3,4-dihydro-2,2-dioxo- 3-methyl- 1,2-benzoxathiin-8-ylsulfonamide of formula I

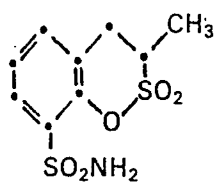
(I),

which comprises reacting a 5-halo-2,3-dihydro-2-methylbenzo[b]furan of formula II

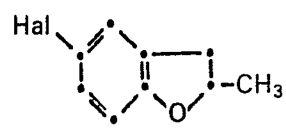
(II),

wherein Hal is chlorine or bromine, with chlorosulfonic acid to give a 6-halo-2,2-dioxo-3-methyl- 1,2-benzoxathiin- 8-ylsulfonyl chloride of formula III

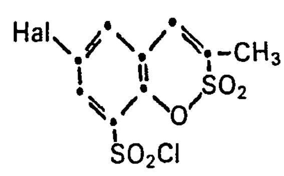
(III),

wherein Hal is chlorine or bromine, converting the sulfonyl chloride with ammonia into a 6-halo-2,2-dioxo- 3-methyl- 1,2-benzoxathiin-8-ylsulfonamide of formula IV

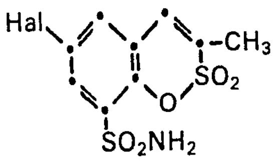

(IV),

wherein Hal is chlorine or bromine, dehalogenating this sulfonamide with hydrogen in the presence of a tertiary amine and a noble metal catalyst, and hydrogenating the resultant 2,2-dioxo-3-methyl-1,2-benzoxathiin- 8-ylsulfonamide of formula V

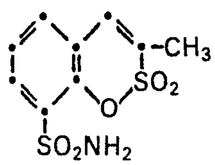

(V)

with hydrogen in the presence of a noble metal catalyst.

2. A process according to claim 1, wherein Hal is chlorine or bromine.

3. A process according to claim 1, wherein the reaction is carried out in all steps in the temperature range from −10 °C to +80 °C, preferably from −10 °C to +60 °C.

4. A process according to claim 1, wherein the individual reaction steps are carried out in inert solvents.

5. A process according to claim 4, which comprises carrying out the reaction of the compound II to give the compound III in methylene chloride, 1,2-dichloroethane or chloroform, the reaction of the compound III to give the compound IV in water, and the reactions of the compound IV to give the compound V and of the compound V to give the compound I in tetrahydrofuran.

6. A process according to claim 1, wherein the reaction of the compound of formula II to give the compound of formula III is carried out with at least 3 moles of chlorosulfonic acid per mole of compound of formula II in an inert solvent and in the temperature range from −10 °C to +80 °C.

7. A process according to claim 6, wherein the reaction of the compound of formula II to give the compound of formula III is carried out with at least 5 moles of chlorosulfonic acid per mole of compound of formula II in the temperature range from −10 °C to +60 °C in methylene chloride, 1,2-dichloroethane or chloroform.

8. A process according to claim 1, wherein the reaction of the compound of formula III to give the compound of formula IV is carried out in an aqueous solution of ammonia.

9. A process according to claim 1, wherein the dehalogenation of the compound of formula IV to give the compound of formula IV is carried out in tetrahydrofuran with hydrogen, under normal pressure at 20 °C to 25 °C and in the presence of a 5% palladium-on-carbon catalyst.

10. A process according to claim 1, wherein the hydrogenation of the compound of formula V to give the compound of formula I is caried out in tetrahydrofuran with hydrogen, in the temperature range from 30 °C to 50 °C under a pressure of 1 to 5 bar and in the presence of a 5% palladium-on-carbon catalyst.

11. A process according to claim 1, which comprises reacting a compound of formula II, wherein Hal is bromine or chlorine, with at least 5 moles of chlorosulfonic acid in the temperature range from −10 °C to +60 °C, in methylene chloride, 1,2-dichloroethane or chloroform, treating the resultant sulfonyl chloride of formula III with an aqueous solution of ammonia, dehalogenating the sulfonamide of formula IV so obtained by catalytic hydrogenation in tetrahydrofuran with hydrogen, under normal pressure in the temperature range from 20 °C to 25 °C and in the presence of a 5% palladium-on-carbon catalyst, and hydrogenating the dehalogenated sulfonamide of formula V in tetrahydrofuran in the temperature range from 30 °C to 50 °C under a pressure of 1 to 5 bar and in the presence of a 5% palladium-on-carbon catalyst.